Europäisches Patentamt

European Patent Office

Office européen des brevets

(11) Publication number: **0 067 041**
**B1**

# EUROPEAN PATENT SPECIFICATION

(45) Date of publication of patent specification: **20.08.86**

(51) Int. Cl.⁴: **G 01 N 33/84**, G 01 N 31/22

(21) Application number: **82302865.9**

(22) Date of filing: **03.06.82**

(54) **Process for measuring calcium.**

(30) Priority: **03.06.81 JP 86021/81**

(43) Date of publication of application:
**15.12.82 Bulletin 82/50**

(45) Publication of the grant of the patent:
**20.08.86 Bulletin 86/34**

(84) Designated Contracting States:
**DE FR IT**

(56) References cited:
**US-A-3 798 000**
**US-A-3 822 116**
**US-A-3 938 954**

**CHEMICAL ABSTRACTS, vol. 67, no. 11, 11th
September 1967, page 4772, col. 1, no. 50967z,
Columbus Ohio (USA); B.C.RAY SARKAR et al.:
"A new method for determining micro
quantities of calcium in biological materials"**

**The file contains technical information
submitted after the application was filed and
not included in this specification**

(73) Proprietor: **WAKO PURE CHEMICAL
INDUSTRIES, LTD.
10, Doshomachi-3-chome·
Higashi-ku Osaka (JP)**

(72) Inventor: **Yamasato, Fujio
3-8-540 Minamisuna-2-chome
Koto-ku Tokyo (JP)**
Inventor: **Tokuda, Kuniaki
515-13 Matoba
Kawagoe-shi (JP)**
Inventor: **Samejima, Kazuhiko
159-1 Oaza Kasahata
Kawagoe-shi (JP)**
Inventor: **Tajima, Hiromi
21-19 Arajukucho-5-chome
Kawagoe-shi (JP)**

(74) Representative: **Baillie, Iain Cameron et al
c/o Ladas & Parry Isartorplatz 5
D-8000 München 2 (DE)**

Courier Press, Leamington Spa, England.

**0 067 041**

(56) References cited:

CHEMICAL ABSTRACTS, vol. 91, no. 1, 2nd July 1979, page 225, col. 2, no. 2180m, Columbus Ohio (USA);

CHEMICAL ABSTRACTS, vol. 93, no. 11, 15th September 1980, page 349, col. 1, no. 109996a, Columbus Ohio (USA); P. CHEUNG: "Methyl alcohol as the stabilizer in a direct colorimetric calcium determination with o-cresolphthalein complexone".

**Description**

This invention relates to a process for measuring the amount of calcium in a sample, living or non-living, such as serum.

Calcium is an inorganic material contained in a living body in the largest amount. On the one hand it together with phosphoric acid forms and maintains bones , it is widely distributed in a living body together with Na and K as one component of an electolyte connected with various important functions. That is, calcium is connected with the control of osmotic pressure of cells and plays a great role in fundamental procedures concerned with the essence of cell functions such as excitation in neuromuscular transporting portions, etc. Thus, the homeostasis is established in a living body mainly via functions of the endocrine system, while the amount of calcium is fixed in a constant narrow concentration range dynamically equilibrated in a body fluid. On the other hand, since the serum calcium is reduced in the case of hypervitaminosis of vitamin D, multiple myelome, acute bone atrophy, tuberculosis of bones and joints, arthritis deformans, sarcoidosis, metastasis of cancer to bones, mild nephritis, hyperparathyroidism, renal rickets, urinemia, last stage nephritis, nephrosclerosis, nephropyelitis, hypoproteinosis, nephrosis, acute pancreatitis, obstructive jaundice, atropic tuberculosis of bones and joints, rickets, pneumonia and whooping cough, the measurement of the amount of calcium is essential for diagnosis of these diseases together with the measurements of serum inorganic phosphorus and alkaline phosphatase. On measuring the amount of calcium, since the changes in amounts are very slight, high precision for the measurement is required.

Among various methods for measuring the amount of calcium such as atomic-absorption spectroscopy, a precipitation method, an EDTA titration method and a direct colorimetric method, a direct colorimetric method using 3,3'-bis[N,N-di(carboxymethyl)-aminomethyl]-ortho-cresolphthalein (usually called ortho-cresolphthalein complexone, hereinafter referred to as "OCPC") as color reagent is presently mainly used. According to said method, color comparison of an OCPC-Ca chelate is conducted at about 570 nm, while masking Mg at pH 10—11 using 8-oxyquinoline or 8-oxyquinoline-5-sulphonic acid. Said method is used widely due to its high sensitivity and relatively low blank value but has the following many disadvantages:

(1) The calibration curve is not straight.

(2) The measured serum value is lower than that of other methods.

(3) Degree of coloring changes greatly depending on temperature, so that strict temperature control is necessary for the measurement.

(4) Since the amount of sample to be measured is very small because of high sensitivity, degree of contamination from test tubes and other apparatus is great.

(5) Boric acid is contained; this is not preferable from the viewpoint of regulations of waste water.

These disadvantages are almost fatal ones to the measurement of calcium which requires high precision as mentioned above. Removal of these disadvantages has long been desired in the field of clinical biochemistry.

In U.S. patent 3 822 116 there is disclosed a process for measuring calcium using OCPC and an amino acid buffer. There is no disclosure of a carbonate buffer. In U.S. patent 3 798 000 there is a disclosed with a different colour reagent a buffer of amidosulfonic acid, disodium tetraborate and an alkali carbonate but there is no indication of a carbonate buffer as in the present invention.

An attempt to remove some of these disadvantages were unsuccessfully tried by the following proposals. For example, Japanese Patent Appln Kokai (Laid-Open) No. 36996/79 proposes the use of a borate buffer solution which belongs to an inorganic system in place of a conventional triethylamine, diethylamine, or monoethanolamine- borate buffer solution so as to improve the differences of degree of coloring due to temperature, and Japanese Patent Appln No. 40555/81 proposes the use of a monoethanolamine-citrate buffer solution so as to improve the linearity of the calibration curve. But these proposals are insufficient to overcome the disadvantages mentioned above.

It is an object of this invention to provide a process for measuring the amount of calcium in a sample overcoming the disadvantages mentioned above.

This invention provide a process for measuring the amount of calcium in a sample using as a color reagent 3,3'-bis [N,N-di(carboxy-methyl)amino-methyl]-ortho-cresolphthalein and a buffer solution, characterized by using a carbonate and/or bicarbonate solution as buffer solution.

In the attached drawings, Fig. 1 is a graph showing an influence of pH using a carbonate buffer solution, Fig. 2 is a graph showing an influence of pH using a monoethanolamine-borate buffer solution, Fig. 3 is a graph showing calibration curves when the pH is changed from 10 to 11 using a carbonate buffer solution, Fig. 4 is a graph showing an influence of pH on calibration curves using a monoethanolamine-borate buffer solution, Fig. 5 is a graph showing an influence of temperature using a carbonate buffer solution, and Fig. 6 is a graph showing the influence of temperature using a monoethanolamine-borate-hydrochloride buffer solution.

In this invention, since a carbonate buffer solution is used in place of conventional buffer solutions containing diethylamine, triethylamine or monoethanolamine and hydrochloric acid or boric acid, the calibration curve becomes linear, the degree of coloring is hardly influenced by temperature changes, and the measured values of serum calcium are in good agreement with those obtained by the atomic-absorption spectroscopy. Although the sensitivity according to the process of this invention is about 40% of that of conventional methods, this makes the present process advantageous by rationally increasing the amount of sample compared with the conventional methods in which a very fine amount of sample is used disregarding sampling precision, which results in making sampling easy and relatively reducing contamination from apparatus and instruments.

The carbonate and/or bicarbonate buffer solution used in this invention can be prepared by blowing carbon dioxide into an aqueous solution of alkali metal hydroxide, or by dissolving in distilled water an alkali metal carbonate or bicarbonate such as lithium carbonate, lithium bicarbonate, sodium carbonate, sodium bicarbonate, potassium carbonate, potassium bicarbonate, or an ammonium salt such as ammonium carbonate or ammonium bicarbonate, alone or as a mixture thereof. The buffer solution is used in combination with a mineral acid such as hydrochloric acid or sulfuric acid, or an organic acid such as acetic acid or citric acid, or an alkali metal hydroxide in order to adjust the pH to the most preferable value. When potassium carbonate or bicarbonate is used in order to suppress contamination by impurities, there can be obtained low reagent blank values. In such case, the carbonate ion concentration is preferably in the range of 0.02 to 0.5 mole/liter.

In the process of this invention, the most suitable pH is about 10.7 as in the case of conventional processes, so that a conventional masking agent for Mg, e.g., 8-hydroxyquinoline or 8-hydroxyquinoline-5-sulfonic acid, can be used.

This invention will be explained referring to the attached drawings.

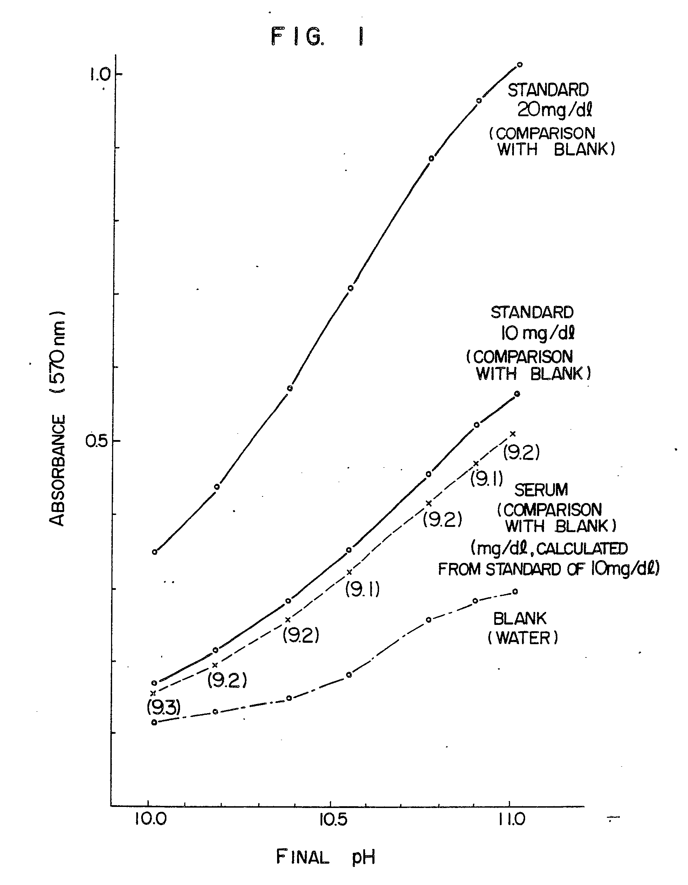

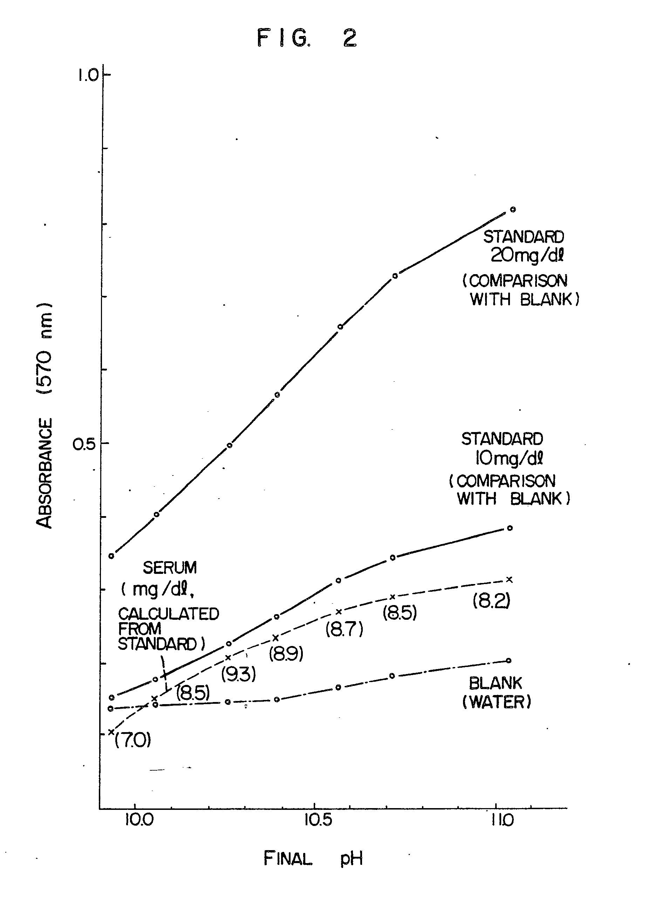

Fig. 1 shows a relationship between pH and sensitivities (absorbance at 570 nm) of blank (water as control), standards (20 mg/dl, 10 mg/dl) (blank, control) and serum (blank, control) (together with concentration, mg/dl, calculated from a standard of 10 mg/dl) using a potassium carbonate and potassium bicarbonate buffer solution according to Example 1 mentioned below. In such a case, the carbonate buffer solution (Reagent solution A) was prepared to give a final pH as shown in the abscissa axis by changing the mixing ratio of potassium carbonate and potassium bicarbonate. Fig. 2 shows a relationship between pH and sensitivities (absorbance at 570 nm) of blank (water as control), standards and serum (in the same manner as described above) using a monoethanolamine-borate buffer solution according to Comparative Example 1 mentioned below. In such a case, the hydrochloric acid concentration in Reagent solution B was adjusted so as to give a final pH as shown in the abscissa axis. According to the conventional buffer solution, the degree of coloring of standard and that of serum do not change in parallel with a change of pH and the measured serum values are different at different pHs, whereas using the carbonate buffer solution, the degrees of coloring of standard and serum change in parallel with a change of pH and the measured serum values do not change depending on the change of pH, although the blank values increase greatly with an increase of pH.

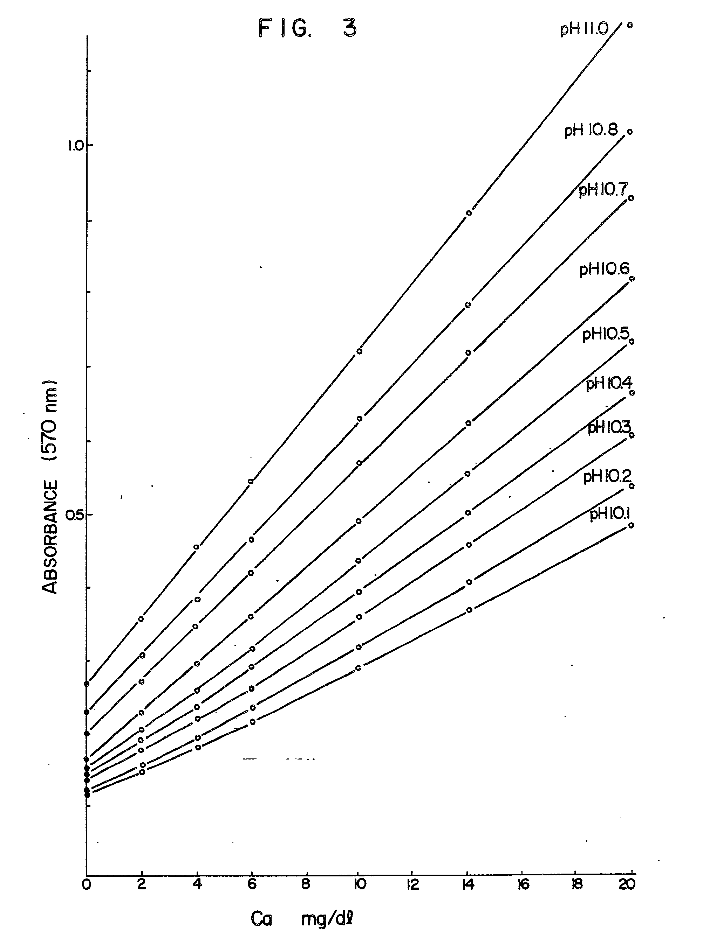

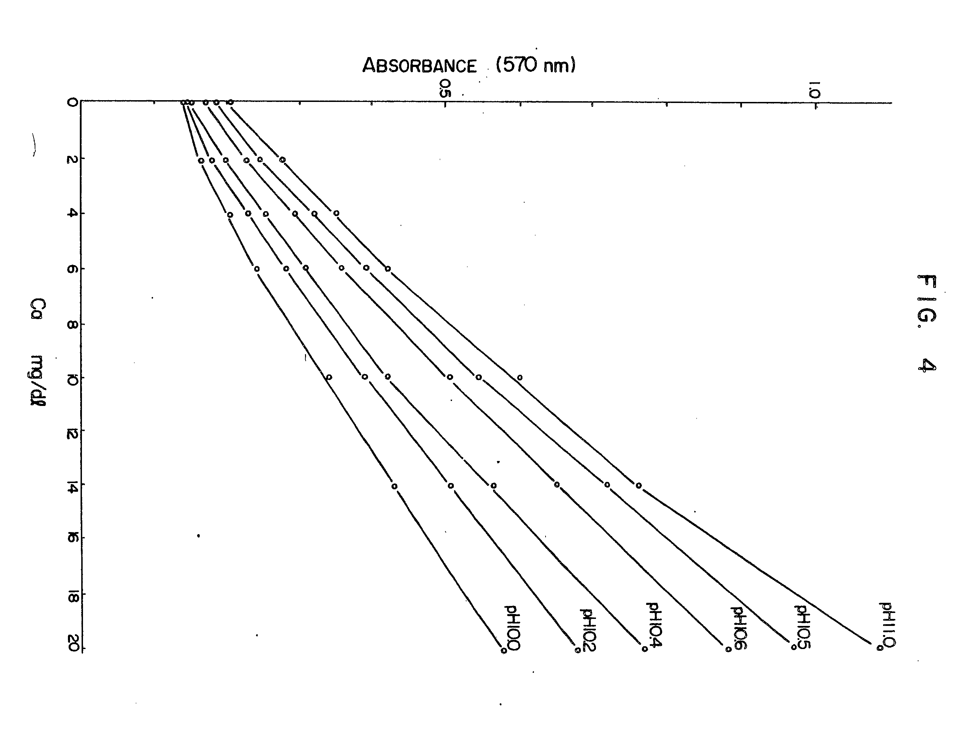

Fig. 3 shows calibration curves of the carbonate buffer solution according to Example 1 when pH is changed from 10 to 11. Fig. 4 shows calibration curves of the monoethanolamine-borate buffer solution according to Comparative Example 1 when pH is changed from 10 to 11. In the case of the carbonate buffer solution, the calibration curves are linear between pH 10.5 and pH 11.0, whereas in the case of the monoethanolamine-borate buffer solution, the calibration curves tend upwardly, not passing an original point.

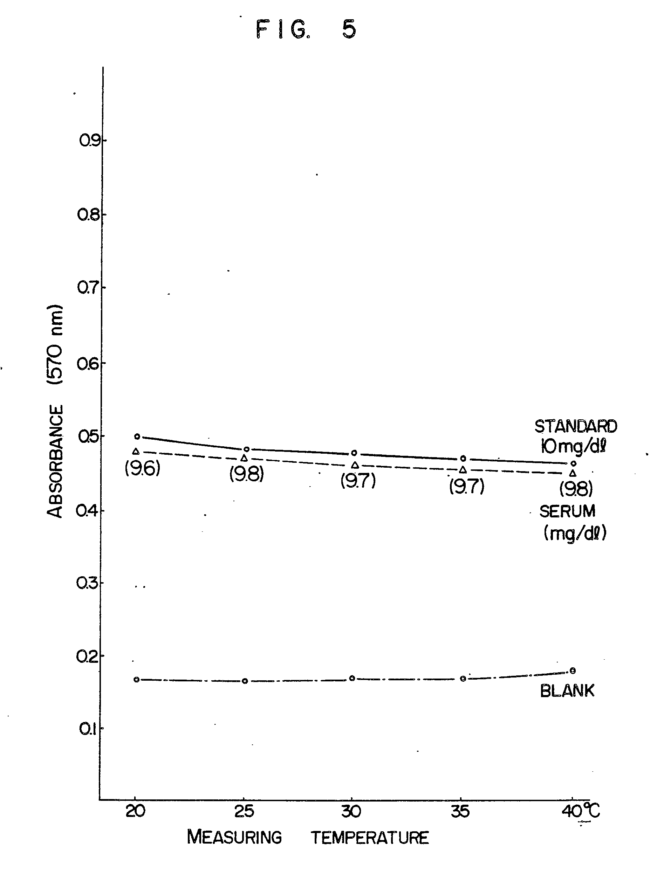

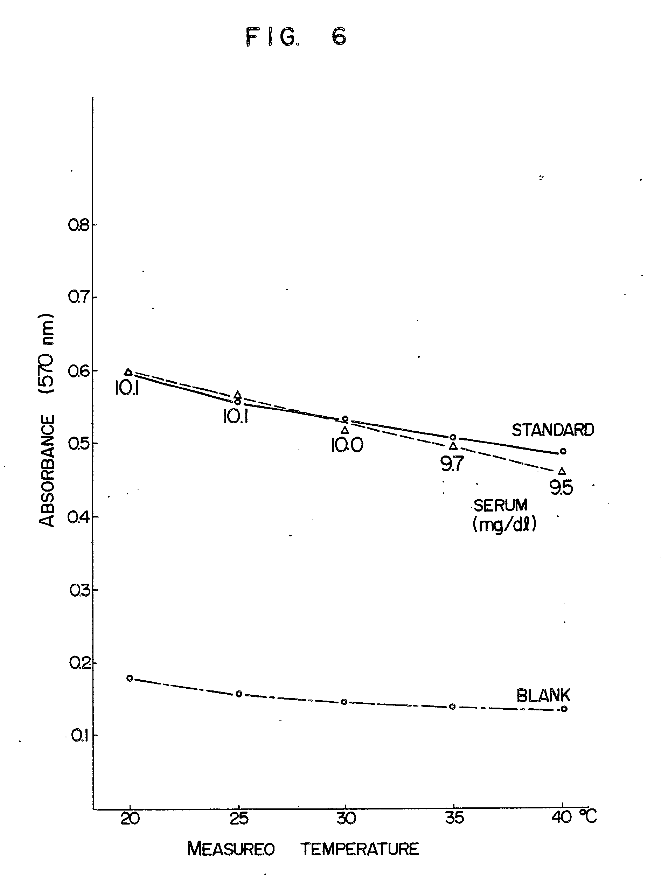

Fig. 5 shows a relationship between degree of coloring (absorbance at 570 nm) of blank (water as control), standard (10 mg/dl) and serum (concentration in mg/dl calculated from the standard) and measuring temperatures changing from 15 to 40°C using the carbonate buffer solution. Fig. 6 shows a relationship between degree of coloring of blank, standard and serum and measuring temperatures changing from 15 to 40°C as mentioned above in the case of the monoethanolamine-borate buffer solution. In the case of the carbonate buffer solution, the blank absorbance slightly increases but absorbances of standard and serum slightly decrease with an increase of the temperature, but the degree of lowering is proportional and the measured serum values are not changed. On the other hand, in the case of the monoethanolamine-borate buffer solution, absorbances of blank, standard and serum are decreased relatively greatly with an increase of the temperature. Further, since the degree of lowering in absorbance in the standard and serum is not parallel with an increase of the temperature and a greater lowering is observed as to the serum, measured serum values are lowered with an increase of the measuring temperature.

Table 1 shows comparison of measured serum values calcium according to the process of this invention, by a conventional method using OCPC, and by an atomic-absorption spectroscopy. The results of Table 1 show that the measured values according to the process of this invention are in good agreement with those obtained by the atomic-absorption spectroscopy, while the measured values according to the conventional method using OCPC and the monoethanolamine-borate buffer solution are lower than those obtained by the atomic-absorption spectroscopy and thus are not precise.

4

TABLE 1

| No. | Process of this invention (mg/dl) | Conventional method using OCPC (mg/dl) | Atomic-absorption spectroscopy (mg/dl) |
|---|---|---|---|
| 1 | 12.1 | 10.7 | 12.3 |
| 2 | 11.0 | 9.5 | 10.8 |
| 3 | 10.5 | 9.1 | 10.7 |
| 4 | 10.2 | 8.7 | 10.5 |
| 5 | 9.6 | 8.2 | 9.4 |
| 6 | 9.5 | 8.1 | 9.2 |
| 7 | 10.6 | 9.2 | 10.9 |
| 8 | 10.4 | 9.0 | 10.5 |
| 9 | 10.3 | 9.0 | 10.3 |
| 10 | 9.8 | 8.4 | 10.1 |
| 11 | 10.4 | 8.9 | 10.4 |
| 12 | 13.2 | 11.6 | 12.8 |
| 13 | 13.1 | 12.3 | 12.9 |
| 14 | 11.5 | 10.1 | 11.8 |
| 15 | 14.9 | 13.7 | 15.0 |
| 16 | 8.5 | 7.2 | 8.7 |
| 17 | 9.1 | 7.6 | 9.2 |
| 18 | 12.9 | 11.2 | 12.8 |
| 19 | 8.0 | 6.7 | 8.3 |
| 20 | 13.2 | 11.7 | 13.4 |
| 21 | 13.7 | 11.9 | 13.9 |
| 22 | 13.6 | 12.3 | 13.6 |
| 23 | 9.4 | 8.4 | 9.5 |
| 24 | 9.3 | 8.0 | 9.4 |
| 25 | 12.7 | 11.0 | 12.9 |
| 26 | 10.8 | 9.7 | 11.0 |
| 27 | 13.5 | 11.9 | 13.6 |
| 28 | 8.2 | 7.3 | 8.3 |
| 29 | 8.4 | 7.2 | 8.4 |
| 30 | 8.7 | 7.5 | 8.8 |
| 31 | 8.9 | 7.6 | 9.2 |

This invention will be explained in more detail by way of the following Examples.

Example 1

Reagent solution A (pH 10.7)

| | |
|---|---|
| Potassium carbonate | 1.76 g |
| Potassium bicarbonate | 0.225 g |

To these compounds, water was added to make a total amount 100 ml.

Reagent solution B

| | |
|---|---|
| OCPC | 4.8 mg |
| 8-Hydroxyquinoline | 160 mg |
| Hydrochloric acid | 83 mg |

To these compounds, water was added to make a total amount 100 ml.

Use Example

To 50 µl of a sample, 1.5 ml of Reagent solution A and 1.5 ml of Reagent solution B were added and allowed to stand at room temperature for 10 minutes. Then, absorbance at 570 nm was measured. The concentration of calcium in the sample was obtained by using calibration curves previously prepared.

Example 2

Reagent solution A (pH 10.7)

| Potassium carbonate | 1.76 g |
|---|---|
| Potassium bicarbonate | 0.225 g |

To these compounds, water was added to make a total amount 100 ml.

Reagent solution B

| OCPC | 4.8 mg |
|---|---|
| 8-Hydroxyquinoline-5-sulphonic acid | 248 mg |

To these compounds, water was added to make a total amount about 90 ml. The pH was adjusted by using sodium hydroxide and hydrochloric acid to pH 5.5 and the total amount was made 100 ml.

Use Example

Equal amounts of Reagent solution A and Reagent solution B were mixed. (The resulting mixture could be used at room temperature for at least 3 days without changing performance.) To 50 µl of a sample, 3.0 ml of the mixed color reagent was added and allowed to stand at room temperature for 10 minutes. Then, absorbance at 570 nm was measured. The concentration of calcium in the sample was obtained by using calibration curves previously prepared.

Comparative Example 1
Conventional OCPC method)

Reagent solution A (pH 11.0)

| Monoethanolamine | 5.37 g |
|---|---|
| Boric acid | 212 mg |

To these compounds, distilled water was added to make a total amount 100 ml.

Reagent solution B

| OCPC | 17 mg |
|---|---|
| 8-Hydroxyquinoline | 400 mg |
| Hydrochloric acid | 700 mg |

To these compounds, distilled water was added to make a total amount of 100 ml.

Use Example

To 20 µl of a sample, 2.0 ml of Reagent solution A and 0.5 ml of Reagent solution B were added and allowed to stand at 20°C for 10 minutes. Then, absorbance at 570 nm was measured. The concentration of calcium in the sample was obtained by using calibration curves previously prepared.

Comparative Example 2
(Atomic-absorption spectroscopy)

Reagent solution A

Trichloroacetic acid in an amount of 4 g was dissolved in water to make a total amount of 100 ml.

Reagent solution B

Strontium chloride in an amount of 3.6 g was dissolved in water to make a total amount 100 ml.

Use Example

To 200 µl of a sample, 1.0 ml of Reagent solution A was added and stirred, followed by addition of 4.0 ml of Reagent solution B. After centrifugal precipitation, the concentration of calcium in the supernatant liquid was measured by using an atomic-absorption spectrometer. Calibration curves were previously

O 067 041

prepared by using physiological saline and standard solutions of calcium in physiological saline. The concentration of calcium in the sample was obtained by using the calibration curves.

As mentioned above, by using the carbonate buffer solution in place of the conventional amine series or borate series buffer solution, disadvantages of the conventional method using OCPC are simultaneously overcome. Further, according to this invention, since no phosphoric acid or boric acid is used, there is no problem in environmental pollution; thus, this invention contributes to clinical chemistry greatly.

**Claims**

1. A process for measuring the amount of calcium in a sample using as a color reagent 3,3'-bis[N,N-di(carboxymethyl)aminomethyl]-orthocresolphthalein and a buffer solution by a colorimetric method, characterized by using a carbonate and/or bicarbonate solution as buffer solution.

2. A process according to Claim 1, wherein the carbonate and/or bicarbonate solution is an aqueous solution of lithium carbonate, lithium bicarbonate, sodium carbonate, sodium bicarbonate, potassium carbonate, potassium bicarbonate, ammonium carbonate, ammonium bicarbonate, or mixtures thereof.

3. A process according to Claim 1 or 2, wherein the carbonate and/or bicarbonate solution is an aqueous solution of potassium carbonate and/or potassium bicarbonate.

4. A process according to Claim 1, wherein the sample is serum.

5. A process according to Claim 1, wherein the colorimetric measurement is conducted at a pH from 10.5 to 11.0.

6. A process according to Claim 1, wherein 8-hydroxyquinoline or 8-hydroxyquinoline-5-sulfonic acid is used as a masking agent for Mg together with the color reagent.

7. A process according to Claim 1 or 2, wherein the carbonate ion concentration in the buffer solution is 0.02 to 0.5 mole/liter.

**Patentansprüche**

1. Kolorimetrisches Verfahren zum Bestimmen des Calcium-gehalts einer Probe mit Hilfe von 3,3'-Bis[N,N-di(carboxymethyl)-aminoethyl]-orthocresolphthalein als Farbreagens und einer Pufferlösung, dadurch gekennzeichnet, daß als Pufferlösung eine Carbonat- oder Bicarbonatlösung verwendet wird.

2. Verfahren nach Anspruch 1, dadurch gekennzeichnet, daß die Carbonat- und/oder Bicarbonatlösung eine wäßrige Lösung von Lithiumcarbonat, Lithiumbicarbonat, Natriumcarbonat, Natriumbicarbonat, Kaliumcarbonat, Kaliumbicarbonat, Ammoniumcarbonat, Ammoniumbicarbonat oder von Gemischen derselben ist.

3. Verfahren nach Anspruch 1 oder 2, dadurch gekennzeichnet, daß die Carbonat- und/oder Bicarbonatlösung eine wäßrige Lösung von Kaliumcarbonat und/oder Kaliumbicarbonat ist.

4. Verfahren nach Anspruch 1, dadurch gekennzeichnet, daß die Probe aus Serum besteht.

5. Verfahren nach Anspruch 1, dadurch gekennzeichnet, daß die kolorimetrische Bestimmung bei einem pH-Wert im Bereich von 10,5 bis 11,0 vorgenommen wird.

6. Verfahren nach Anspruch 1, dadurch gekennzeichnet, daß zusammen mit dem Farbreagens als Maskierungsmittel für Mg 8-Hydroxychinolin oder 8-Hydroxychinolin-5-sulfonsäure verwendet wird.

7. Verfahren nach Anspruch 1 oder 2, dadurch gekennzeichnet, daß die Carbonationenkonzentration in der Pufferlösung 0,02 bis 0,5 Mol/l beträgt.

**Revendications**

1. Procédé pour mesurer la quantité de calcium dans un échantillon en utilisant comme réactif coloré la 3,3'-bis[N,N-di(carboxyméthyl)aminométhyl]-orthocrésolphthaléine et une solution tampon par une méthode colorimétrique, caractérisé en ce qu'on utilise comme solution tampon une solution de carbonate et/ou de bicarbonate.

2. Procédé suivant la revendication 1, dans lequel la solution de carbonate et/ou de bicarbonate est une solution aqueuse de carbonate de lithium, de bicarbonate de lithium, de carbonate de sodium, de bicarbonate de sodium, de carbonate de potassium, de bicarbonate de potassium, de carbonate d'ammonium, de bicarbonate d'ammonium ou de mélanges de ceux-ci.

3. Procédé suivant la revendications 1 ou 2, dans lequel la solution de carbonate et/ou de bicarbonate est une solution aqueuse de carbonate depotassium et/ou de bicarbonate de potassium.

4. Procédé suivant la revendication 1, dans lequel l'échantillon est du sérum.

5. Procédé suivant la revendication 1, dans lequel la mesure colorimétrique est effectuée à un pH de 10,5 à 11,0.

6. Procédé suivant la revendication 1, dans lequel la 8-hydroxyquinoléine ou l'acide 8-hydroxyquinoléine-5-sulfonique sont utilisés comme agent de masquage pour le Mg en même temps que le réactif coloré.

7. Procédé suivant la revendications 1 ou 2, dans lequel la concentration en ion carbonate dans la solution tampon est de 0,02 à 0,5 mole/litre.

7

FIG. 1

STANDARD
20mg/dl
(COMPARISON
WITH BLANK)

STANDARD
10 mg/dl
(COMPARISON
WITH BLANK)

(9.2)

(9.1)

SERUM
(COMPARISON
WITH BLANK)
(mg/dl, CALCULATED
FROM STANDARD OF 10mg/dl)

(9.2)

(9.1)

(9.2)

BLANK
(WATER)

(9.2)

(9.3)

ABSORBANCE (570 nm)

1.0

0.5

10.0        10.5        11.0

FINAL pH

FIG. 2

FIG. 3

F I G. 4

FIG. 5

FIG. 6